# EUROPEAN PATENT APPLICATION

(11) **EP 4 556 551 A1**
(43) Date of publication of application: **21.05.2025**
(21) Application number: 23839593.3
(22) Date of filing: 10.07.2023
(51) Int. Cl.: C12M 1/02, C12M 1/26

(54) **DISPENSING PREPARATION DEVICE, DISPENSING DEVICE, AND DISPENSING PREPARATION METHOD**

(30) Priority: 12.07.2022 JP 2022111714
(71) Applicant: Cyfuse Biomedical K. K., Tokyo 108-6301 (JP)
(72) Inventor: KISHII, Yasuto, Tokyo 108-6301 (JP); TOKUNAGA, Norihiko, Tokyo 108-6301 (JP)
(74) Representative: Grünecker Patent- und Rechtsanwälte PartG mbB
(86) International application number: PCT/JP2023/025380
(87) International publication number: WO 2024/014421

(57) **Abstract**

A dispensing preparation apparatus capable of stirring cell suspension so as not to damage cells is desired. It is solved by the dispensing preparation apparatus including a reservoir configured to accommodate cell suspension, the reservoir having an opening, a discharge tip portion of a pipette repeatedly entering and exiting through the opening, the pipette discharging the cell suspension to a cell accommodating portion, a tank configured to accommodate the cell suspension, a pipe connecting the tank and the reservoir, the pipe configured to allow the cell suspension to flow inside from the tank to the reservoir by a pump unit, a turntable configured to rotate the tank about a rotation axis perpendicular to a bottom surface of the tank, and a controller configured to control rotation of the turntable, the pipe arranged such that an opening at one end of the pipe contacts an inner wall of the reservoir and an opening at another end of the pipe is spaced apart from an inner wall of the tank.

## Description

### Technical Field

The present invention relates to a dispensing preparation apparatus of cell suspension for dispensing cell suspension, a dispensing apparatus using the dispensing preparation apparatus, and a dispensing preparation method.

### Background Art

In culturing cells to create spheroids or the like formed by aggregating single cells, the cells to be cultured are placed in each well of a well plate and cultured in a CO₂ incubator. In the preliminary stage, it is necessary to dispense cell suspension containing the cells to be aggregated into each well of the well plate.

For example, PTL 1 and PTL 2 disclose dispensing apparatuses in which a cell suspension reservoir is directly connected toward a pipette for dispensing cell suspension into a plate, and that can continuously repeat dispensing of the cell suspension into plates, and associated apparatuses.

### Citation List

### Patent Literature

PTL 1: Japanese Patent No. 6906773
PTL 2: Japanese Patent Application Laid-Open No. 2021-000018

### Summary of Invention

### Technical Problem

When automating a process of dispensing cell suspension containing cells to be cultured into each well, it is necessary that the cells are uniformly contained in the suspension to be dispensed into each well, and that the cells are not damaged. In this regard, PTL 1 discloses neither that the cells are evenly and uniformly distributed in the suspension, nor that the cells are not to be damaged at the stage of dispensing. In addition, in the dispensing apparatus disclosed in PTL 2, although reference is made to a suspension stirring apparatus, a countermeasure for preventing cells from being damaged is not disclosed.

In general, not only for cells, but throughout the entire area of a medium containing matters, although a typical operation for homogenizing the matters is "stirring," "stirring" involves the flow of the matters within the medium. Therefore, for homogenization of cells in suspension containing the cells, the application of stirring may cause direct damage to the cells. Therefore, it is difficult to simply apply "stirring" to a step of dispensing cells. Accordingly, a dispensing apparatus capable of stirring cell suspension without damaging cells is desired.

### Solution to Problem

An aspect of the present invention is a dispensing preparation apparatus including a reservoir configured to accommodate cell suspension, the reservoir having an opening, a discharge tip portion of a pipette repeatedly entering and exiting through the opening, the pipette discharging the cell suspension to a cell accommodating portion, a tank configured to accommodate the cell suspension, a pipe connecting the tank and the reservoir, the pipe configured to allow the cell suspension to flow inside from the tank to the reservoir by a pump unit, a turntable configured to rotate the tank about a rotation axis perpendicular to a bottom surface of the tank, and a controller configured to control rotation of the turntable, the pipe arranged such that an opening at one end of the pipe contacts an inner wall of the reservoir and an opening at another end of the pipe is spaced apart from an inner wall of the tank, the controller controlling initial stirring to be performed, the initial stirring including initial inertial flow generation of the cell suspension followed by reverse flow generation, the initial inertial flow generation involving forward rotation of the turntable in a predetermined direction about the rotation axis a first number of times, the reverse flow generation involving reverse rotation of the turntable in a direction opposite to the predetermined direction a second number of times.

Another aspect of the present invention is a dispensing preparation method of stirring cell suspension in a tank by a dispensing preparation apparatus, the dispensing preparation apparatus including a reservoir configured to accommodate the cell suspension, the reservoir having an opening, a discharge tip portion of a pipette repeatedly entering and exiting through the opening, the pipette discharging the cell suspension to a cell accommodating portion, the tank configured to accommodate the cell suspension, a pipe connecting the tank and the reservoir, the pipe configured to allow the cell suspension to flow inside from the tank to the reservoir by a pump unit, and a turntable configured to rotate the tank about a rotation axis perpendicular to a bottom surface of the tank, the dispensing preparation method including a step of performing initial stirring, the initial stirring including initial inertial flow generation of the cell suspension followed by reverse flow generation, the initial inertial flow generation involving forward rotation of the turntable in a predetermined direction about the rotation axis a first number of times, the reverse flow generation involving reverse rotation of the turntable in a direction opposite to the predetermined direction a second number of times.

### Advantageous Effects of Invention

According to the present invention, it becomes possible to manage and dispense suspension to be dispensed so that cells are evenly and uniformly contained in the suspension without damaging the cells.

### Brief Description of Drawings

FIG. 1 illustrates an external configuration of a dispensing preparation apparatus, which is an embodiment of the present invention.
FIG. 2 illustrates a functional block diagram of the dispensing preparation apparatus and a dispensing apparatus, which are embodiments of the present invention.
FIG. 3 illustrates an external configuration of a dispensing apparatus, which is an embodiment of the present invention.
FIG. 4 illustrates a conceptual cross-section of the relationship between a front end of a pipette and a reservoir in the dispensing preparation apparatus, which is an implementation of the present invention.
FIG. 5 illustrates a conceptual cross-section of the relationship between the front end of the pipette and a suspension liquid level inside a well in the dispensing preparation apparatus, which is the implementation of the present invention.

### Description of Embodiments

### (Embodiments of the present invention)

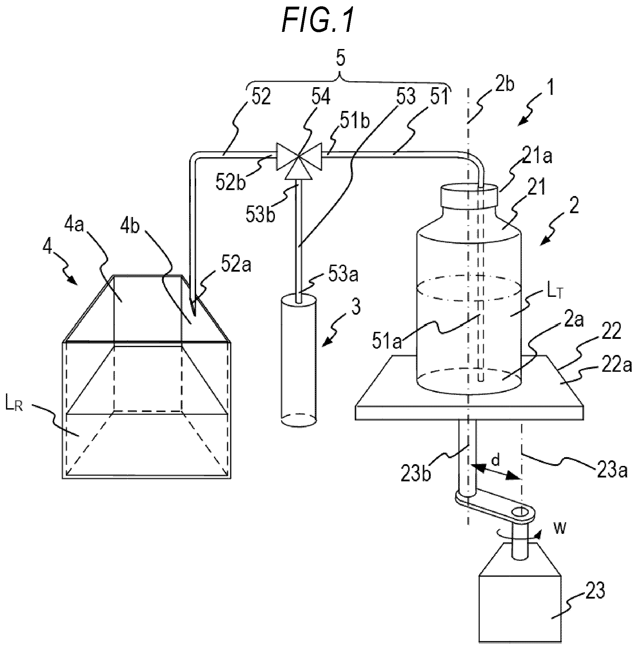

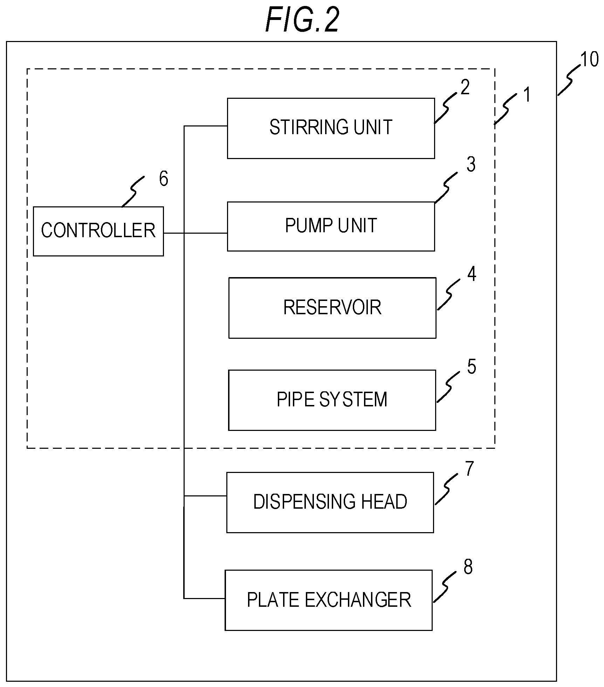

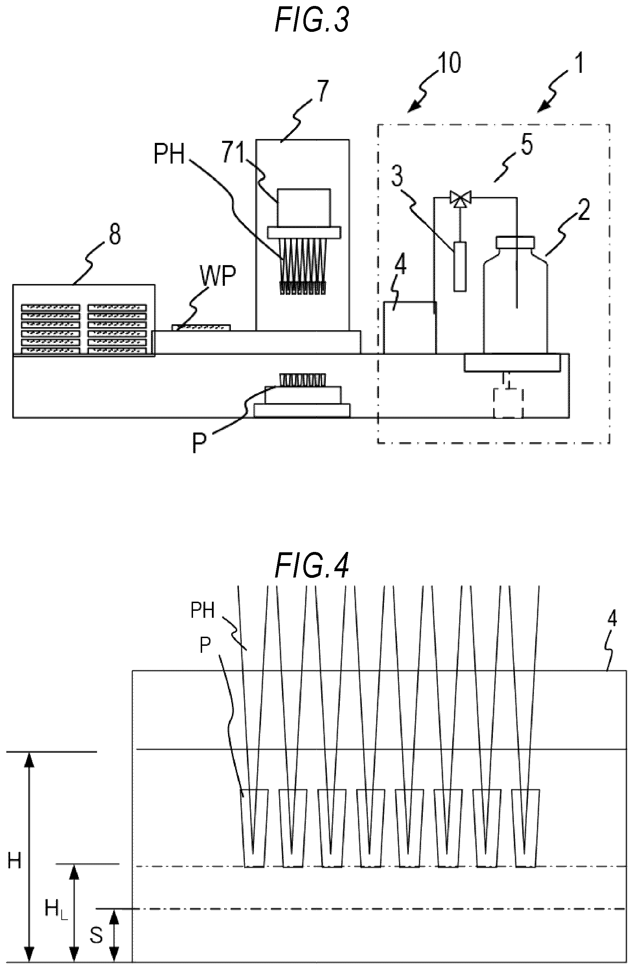

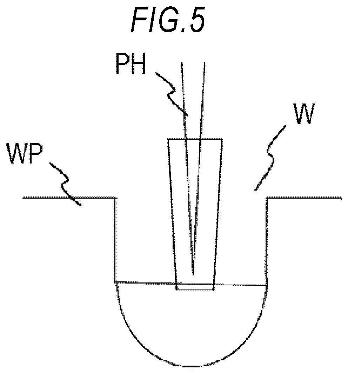

Referring to FIG. 1 to FIG. 5, a description will be given of a dispensing preparation apparatus 1 and a dispensing apparatus 10 according to embodiments of the present invention. FIG. 1 illustrates the external configuration of the dispensing preparation apparatus 1. FIG. 2 illustrates a functional block diagram of the dispensing preparation apparatus 1 and the dispensing apparatus 10. FIG. 3 illustrates the external configuration of the dispensing apparatus 10. FIG. 4 illustrates the relationship between a front end of a pipette and a suspension liquid level inside a reservoir 4 of the dispensing preparation apparatus 1. FIG. 5 illustrates a concept of the relationship between the front end of the pipette and the suspension liquid level inside a well in the dispensing preparation apparatus 1.

### [Example 1]

### (Dispensing preparation apparatus and dispensing apparatus)

The dispensing preparation apparatus 1 includes a bottle tank (hereinafter, "the tank") 2, a turntable 22, the reservoir 4, a pipe system 5, and a controller 6. The dispensing preparation apparatus 1 can be combined to constitute a part of the dispensing apparatus 10. For example, the dispensing apparatus 10 can be configured to include, for example, a dispensing head 7 and a plate exchanger 8, in addition to the dispensing preparation apparatus 1.

The dispensing head 7 causes a pipette with a pipet chip attached to a front end to enter and exit the inside of the reservoir 4, to accommodate cell suspension therein. Then, the cell suspension is poured into a cell accommodating portion of a container having the cell accommodating portion. The container having the cell accommodating portion is, for example, a well plate, and the cell accommodating portion is each of wells thereof. The dispensing head 7 pours the cell suspension into a well by the pipette.

When the dispensing head 7 sucks the cell suspension from the reservoir 4, the dispensing head 7 causes the pipette to enter the inside of the reservoir 4. At this time, the suction of the cell suspension is started after the front end of the pipet chip reaches at least a 1/3 depth of the depth in a direction of depth H of the cell suspension from the surface of cell suspension in the reservoir 4, preferably a predetermined depth H_{L}, i.e., a 1/2 depth. After the suction of a predetermined amount of the cell suspension is completed, the pipette is caused to exit the inside of the reservoir 4. When the depth H of the cell suspension in the reservoir 4 reaches a predetermined critical depth S, the dispensing head 7 does not cause the pipette to enter and exit the inside of the reservoir 4. The critical depth S can be freely set within the range where the front end of the pipette tip does not contact a bottom of the reservoir 4, in consideration of manufacturing tolerances of the pipet chip and the pipette, the accuracy of the moving distance of the dispensing head, variation in the amount of the cells in the residual liquid of the cell suspension in the reservoir, and the like.

When the pipette pours the cell suspension into the cell accommodating portion, the dispensing head 7 does not pour the cell suspension while the front end of the pipette penetrates the inside of the cell accommodating portion. After the front end of the pipette completely penetrates the inside of the cell accommodating portion, the dispensing head 7 starts exiting the inside of the cell accommodating portion. After the pipette starts exiting the inside of the cell accommodating portion, the pipette pours the cell suspension into the cell accommodating portion.

The plate exchanger 8 moves a container in which the cell suspension has not been poured to the cell accommodating portion to a predetermined location. There, the dispensing head 7 pours the cell suspension into the cell accommodating portion by the pipette.

The plate exchanger 8 collects the container in which the cell suspension has been poured into the cell accommodating portion.

### (Dispensing preparation apparatus)

The reservoir 4 has an opening 4a, and accommodates cell suspension L_{R} inside. A discharge tip portion of the pipette, which discharges the cell suspension to the cell accommodating portion, repeatedly enters and exits through the opening 4a.

A tank 2 is arranged beside the reservoir 4. The tank 2 consists of a bottle container 21 that has an opening at one end and that is hollow inside, and a cap 21a capable of closing the opening of the bottle container 21. The bottle container 21 has, for example, a bottom surface 2a, and has a cylinder wall portion extending in a vertical direction along a tank axis 2b that passes through the center of the bottom surface 2a and that extends perpendicularly to the bottom surface 2a. A hollow portion is defined inside by the cylinder wall portion and the bottom surface 2a. Cell suspension L_{T} is accommodated inside the tank 2. The center of the bottom surface 2a of the bottle container 21 of the tank 2 is, for example, the center of gravity. The tank 2 typically has a cylindrical shape with the bottom surface 2a having a circular shape, and the tank axis 2b is an axis passing through the center of the bottom surface 2a. The bottom surface 2a of the tank 2 is not limited to a circular shape, and may be a polygon including a rectangle or a long round shape such as an oval shape. Also in this case, a main body has a hollow shape extending along the direction of the tank axis 2b perpendicular to the bottom surface 2a. In this description, the tank 2 will be described as having a hollow cylindrical shape with the circular bottom surface 2a. The tank 2 can accommodate, in this inner hollow portion, cell suspension in which a predetermined amount of cells is mixed into a predetermined culture medium.

The pipe system 5 connects the tank 2 and the reservoir 4. A pump unit 3 is connected to the pipe system 5. The pump unit 3 is, for example, a reciprocating pump having a cylinder inside. The pipe system 5 consists of a tank pipe 51 whose one end 51a is connected to the tank 2, a reservoir pipe 52 whose one end 52a is connected to the reservoir 4, a pump pipe 53 whose one end 53a is connected to the pump unit 3, and a valve 54. The valve 54 is a three-way valve, and the other end 51b of the tank pipe 51, the other end 52b of the reservoir pipe 52, and the other end 53b of the pump pipe 53 are connected to the valve 54. The valve 54 can be, for example, a magnetic valve or a rotary valve. The valve 54 can be switched between a first state of closing connection with the reservoir pipe 52 and connecting the tank pipe 51 and the pump pipe 53, and a second state of closing connection with the tank pipe 51 and connecting the reservoir pipe 52 and the pump pipe 53.

Then, in a case where the valve 54 is switched to the first state, when the pump unit 3 retracts the cylinder to sucks the inside, the pump pipe 53 and the tank pipe 51 is under negative pressure, and the cell suspension in the tank 2 flows into the pump pipe 53 and the tank pipe 51. Subsequently, in a case where the valve 54 is switched to the second state, when the pump unit 3 advances the cylinder to increase the pressure inside, the cell suspension inside the pump pipe 53 is sent out to the reservoir pipe 52, and finally, the cell suspension inside the pump pipe 53 is transferred into the reservoir 4. Accordingly, the cell suspension is transferred from the tank 2 to the reservoir 4 via the tank pipe 51 and the reservoir pipe 52. The tank pipe 51, the pump pipe 53, and the reservoir pipe 52 suck the cell suspension in the tank 2, and the pump pipe 53 and the reservoir pipe 52 are filled with the cell suspension. In this example, the valve 54 sets the pipe system to the first state as the cylinder of the pump unit 3 is retracted, and the valve 54 sets the pipe system to the second state as the cylinder of the pump unit 3 advances.

The one end 52a of the reservoir pipe 52, which is an opening at one end of the pipe system 5, is arranged inside the reservoir 4 to contact an inner wall of the reservoir **4.** In addition, the one end 51a of the tank pipe 51, which is an opening at the other end of the pipe system 5, is supported by a holding member (not illustrated), to be spaced apart from an inner wall of the tank 2, and so that the one end 51a of the tank pipe 51 is located in the cell suspension L_{T} in the tank 2. The tank pipe 51 is fixed above the opening of the tank 2, and the one end 51a of the tank pipe 51 is inserted into the inside of the tank 2 from the opening of the tank 2. The reservoir 4 has a liquid level detection sensor (not illustrated) to detect the liquid level height, and is capable of detecting the liquid level height of the cell suspension inside the reservoir 4. The liquid level detection sensor (not illustrated) is connected to the controller 6. When the liquid level detection sensor (not illustrated) detects the liquid level height of the reservoir 4, the liquid level height is notified to the controller 6, and in a case where the controller 6 determines that the liquid level height of the liquid level detection sensor is less than a predetermined height, the controller 6 issues an operational instruction to the pump unit 3 so that the pump unit 3 is operated. The pump unit 3 that has received the operational instruction from the controller 6 supplies the cell suspension from the tank 2 to the reservoir 4 through the pipe system 5 as described above. The liquid level detection sensor (not illustrated) can be of a type other than a type of directly detecting the height of the liquid level. For example, the height of the liquid level can be indirectly obtained by calculating the height of the liquid level from the amount of transfer of the cell suspension to the reservoir 4. That is, as described above, the amount of the cell suspension sent out by the pump unit 3 corresponds to the decrease in the liquid amount in the tank 2 and the increase in the liquid amount in the reservoir 4. As in this embodiment, particularly in the case where the pump unit 3 is a reciprocating pump, the amount of the cell suspension sent out by the pump unit 3 directly corresponds to the volume of the cylinder of the pump unit 3.

The dispensing preparation apparatus 1 includes a turntable 22 having a placement surface 22a that is a substantially horizontal surface rotated by a motor 23. The turntable 22 is rotated about a shaft 23b extending along a vertical rotation axis 23a that extends perpendicularly to the bottom surface 2a of the tank 2. The motor 23 rotates the vertical rotation axis 23a. The vertical rotation axis 23a is the rotation center of a motor, and the tank 2 can be placed on the placement surface 22a of the turntable 22 to have the center on the tank axis 2b, which is the axis eccentric with respect to the vertical rotation axis 23a by the distance of a radius of rotation d. The turntable 22 can rotate the tank 2 placed on the placement surface 22a of the turntable 22 about the vertical rotation axis 23a. The tank 2 placed on the placement surface 22a of the turntable 22 is rotated in a predetermined direction by the rotation of the turntable 22 around the vertical rotation axis 23a. The rotation of the turntable 22 is rotation defined by predetermined rotation conditions, such as the predetermined radius of rotation d, a predetermined angular velocity w, and a predetermined angular acceleration. The tank pipe 51 is inserted into the tank 2, and is held so that the position of the one end 51a of the tank pipe 51 is not changed and twisted even if the tank 2 is rotated by the turntable 22. For example, it is possible to adopt a structure in which the cap 21a of the tank 2 can have a hole through which the tank pipe 51 is inserted, the tank pipe 51 is inserted into the inside of the tank 2 from the hole, and the cap 21a holds the tank pipe 51. Accordingly, particularly, even in a case where the radius of rotation of the turntable 22 is large, the tank pipe 51 is not twisted, and even in a case where the tank 2 is rotated by the turntable 22, the position of the one end 51a of the tank pipe 51 is not changed, and the load due to twisting is not applied to the tank pipe 51. The tank pipe 51 may be configured as the tank pipe 51 that is divided into two members, one inside and one outside the tank 2, at a cap portion, the two members being connected by the cap to connect the inside and outside of the tank 2.

The radius of rotation d of the tank 2 at this time is defined as the distance between the tank axis 2b of the tank 2 and the vertical rotation axis 23a of the turntable 22. For example, the radius of rotation d of the tank 2 can be equal to or less than the diameter of an inscribed circle of the bottom surface 2a of the tank 2. In a case where the bottom surface 2a of the tank 2 is a perfect circle, the radius of rotation d of the tank 2 is preferably equal to or less than the diameter of the bottom surface 2a of the tank 2. In addition, in a case where the bottom surface 2a of the tank 2 is a perfect circle, the radius of rotation d of the tank 2 is more preferably 1.2 times the radius of the bottom surface 2a of the tank 2. However, the radius of rotation d of the tank 2 is not limited to these, and can be set freely, as long as the tank 2 can be rotated about the rotation axis perpendicular to the bottom surface of the tank 2.

The controller 6 controls the rotation of the turntable 22. In a case where the tank 2 is placed on the placement surface 22a of the turntable 22, the controller 6 rotates the turntable 22 to perform initial stirring of cells in the cell suspension via the rotation of the tank 2. The initial stirring is constituted by initial inertial flow generation (forward rotation), and reverse flow generation (reverse rotation) executed following the initial inertial flow generation (forward rotation). Only with the initial inertial flow generation (forward rotation), the cells simply rotate in the swirling flow of the cell suspension in the tank 2, and no stirring effect is produced. Therefore, the initial stirring is only effective with the initial inertial flow generation (forward rotation) and the reverse flow generation (reverse rotation) executed following the initial inertial flow generation (forward rotation). That is, the controller 6 controls the turntable 22, to perform the initial inertial flow generation about the vertical rotation axis 23a in a predetermined direction of the vertical rotation axis 23a, and then to perform rotation about the vertical rotation axis 23a in the direction opposite to the direction of the initial inertial flow generation. In this description, the direction of the initial inertial flow generation is defined as the forward rotation. The rotation direction of the initial inertial flow generation (forward rotation) can be arbitrarily set according to the angular velocity of the rotation of the turntable 22. That is, the controller 6 rotates the turntable 22 by combining the initial inertial flow generation (forward rotation) and the reverse flow generation (reverse rotation). This initial stirring (reverse rotation) creates a stirring effect in the cell suspension as a flow that counteracts the initial inertial flow generation (forward rotation) generated in the tank 2. That is, the initial stirring (reverse rotation) immediately after the initial inertial flow generation (forward rotation) may be in the direction opposite to the rotation direction of the initial inertial flow generation (forward rotation). The initial inertial flow generation (forward rotation) is performed a first number of times, and the reverse flow generation (reverse rotation) is performed a second number of times. The second number of times is equal to or less than the first number of times, and typically, the second number of times is less than the first number of times.

The predetermined angular velocity w of the turntable 22 by the controller 6 is defined as an angular velocity within a range in which a stirring effect of cells in the cell suspension is produced and within a range in which the cells are not damaged. While the effect of stirring of cells in the cell suspension is reduced in a case where the angular velocity of the rotation is too slow, the cells in the cell suspension may be damaged due to stirring in a case where the angular velocity of the rotation is too fast. The predetermined angular velocity w is, for example, about 2 rotations per second (2 rps).

In addition, the reverse flow generation (reverse rotation) of the initial stirring has, in addition to the stirring effect in the cell suspension, an effect of counteracting the stirring up of the cell suspension generated by the initial inertial flow generation (forward rotation). That is, since the reverse flow generation (reverse rotation) is the flow in the opposite direction of the initial inertial flow generation (forward rotation), while spiral stirring up is generated in the cell suspension in the tank 2 due to the initial inertial flow generation (forward rotation), the stirring up of the cell suspension generated inside the tank 2 is canceled by the reverse flow generation (reverse rotation) to prevent the cell suspension from churning inside the tank 2.

For example, as the initial stirring, the controller 6 first performs the initial inertial flow generation (forward rotation) for seven rotations of the turntable 22, and thereafter performs the reverse flow generation (reverse rotation) for five rotations of the turntable 22. For example, typically, the turntable 22 is rotated seven to ten times as the initial inertial flow generation (forward rotation), and the turntable 22 is rotated five to seven times as the reverse flow generation (reverse rotation). With the initial inertial flow generation (forward rotation) of seven to ten rotations and the reverse flow generation (reverse rotation) of five to seven rotations, the stirring effect and the cancellation effect of stirring up in three directions, i.e., the radial direction, the circumferential direction, and the depth direction of the tank 2, can be realized in the tank 2. The former stirring effect is an effect on the uniform distribution of cells in the cell suspension, and the latter cancellation effect of stirring up is an effect on the prevention of cells from being damaged by the flow of the cell suspension due to stirring, in addition to the former stirring effect.

Although stirring up occurs in the cell suspension in a case where the turntable 22 is rotated seven to ten times (first rotation) as the initial inertial flow generation (forward rotation), the stirring up can be cancelled by performing five to seven rotations (second rotation) as the reverse flow generation (reverse rotation). For the cancellation of stirring up**,** it is preferable that the number of times of rotation is less than the number of times of rotation that has generated the flow of stirring up, particularly about 70 percent of the number of rotations. As described above, since the rotation (second rotation) of the reverse flow generation (reverse rotation) plays a role of canceling the stirring up, the reverse flow generation (reverse rotation) is set to equal to or less than the rotation (first rotation) of the initial inertial flow generation (forward rotation). In addition, typically, it is particularly preferable to set the reverse flow generation (reverse rotation) to less than the rotation (first rotation) of the initial inertial flow generation (forward rotation). For example, it is preferable to set the reverse flow generation (reverse rotation) to approximately 70 percent of the rotation of the initial inertial flow generation (forward rotation).

Furthermore, at least one additional stirring can be added and executed after the initial stirring. The number of times of additional stirring can be freely set, depending on the distribution state of cells in the cell suspension. One additional stirring consists of a combination of additional inertial flow generation (forward direction) and additional reverse flow generation (reverse direction) of the turntable 22. That is, one additional stirring consists of, after the reverse flow generation (reverse rotation) of the initial stirring, the additional inertial flow generation (forward rotation) and the additional reverse flow generation (reverse rotation) after the additional inertial flow generation (forward rotation) of the turntable 22. The number of times of rotation of the additional inertial flow generation (forward rotation) and the number of times of rotation of the additional reverse flow generation (reverse rotation) can be freely set depending on, for example, the state of cells in the tank 2, such as the distribution state of cells in the cell suspension. Furthermore, additional stirring can also be performed a plurality of times. In this case, after the additional reverse flow generation (reverse rotation) of the previous additional stirring, additional stirring consisting of additional forward rotation and additional reverse rotation after the additional forward rotation of the turntable 22 may further be performed. Generally, the number of times of rotation of the additional reverse flow generation (reverse rotation) is equal to or less than the number of times of rotation of the additional inertial flow generation (forward rotation). For example, in one additional stirring, the number of times of rotation of the additional reverse flow generation (reverse rotation) is set to two times, and the number of times of rotation of the additional inertial flow generation (forward rotation) is set to two times or one time. The additional reverse flow generation (reverse rotation) contributes to the effect of cancelling the inertia of the flow leading to stirring up by the additional inertia style generation (forward rotation) as well as the effect of stirring cells in the suspension.

Additional stirring can be performed, for example, a plurality of times. For example, in an example where additional stirring is performed two times, a first additional stirring and a second additional stirring after the first additional stirring are performed. In the first additional stirring, first additional inertial flow generation (forward direction) and first additional reverse flow generation (reverse direction) following the first additional inertial flow generation (forward direction) are performed. In the second additional stirring, second additional inertial flow generation (forward direction) after the first additional reverse flow generation (reverse direction) and second additional reverse flow generation (reverse direction) following the second additional inertial flow generation (forward direction) are performed. In the first additional stirring and the second additional stirring, the number of times of rotation of each additional reverse flow generation (reverse rotation) is set to two times, and the number of times of rotation of the additional inertial flow generation (forward rotation) can be freely set as needed.

In additional stirring, for example, in one additional stirring, the number of times of rotation of additional reverse flow generation (reverse rotation) can be set to two times, and the number of times of rotation of additional inertial flow generation (forward rotation) can be set to two times, and such additional stirring can be performed five times. That is, for example, after initial stirring in which the turntable 22 is rotated seven to ten times as the initial inertial flow generation (forward rotation), and the turntable 22 is rotated five to seven times as the initial stirring (reverse rotation), one additional stirring consisting of two times of additional forward rotation and two times of additional reverse rotation is consecutively performed five times. Accordingly, the distribution of the cells is uniform in the cell suspension in the three directions inside the tank 2, i.e., the radial direction, the circumferential direction, and the depth direction of the tank 2.

In a case where the cell suspension is stirred to make the distribution of the cells contained in the cell suspension uniform within the cell suspension, if stirring is performed by rotation in only one direction, the inertial flow of the cell suspension is generated and the cells are simply rotated together with the cell suspension, and the cells contained in the cell suspension cannot be made uniform within the cell suspension. Therefore, as described above, in each of the initial stirring and the additional stirring, after generating the inertial flow by rotation in a direction of the forward rotation, the reverse flow generation is performed by performing rotation in the reverse direction.

Furthermore, in a case where rotational flow of the cell suspension is generated due to stirring, a whirlpool is generated in the cell suspension. When the whirlpool is generated in the cell suspension, after stirring ends, cells will gather at the center of the tank 2 due to the whirlpool inside the cell suspension. An appropriate number of times of additional stirring has an effect of cancelling the whirlpool generated due to the stirring flow. Therefore, in the additional stirring, it is preferable to set the number of times and the number of times of rotation to be gradually decreased. That is, each rotation in the additional inertial flow generation (forward rotation) and the additional reverse flow generation (reverse rotation) constituting the additional stirring is set to be less than each rotation in the initial inertial flow generation and the reverse flow generation constituting the initial stirring.

This application claims priority from Japanese Patent Application No. 2022-111714 filed on July 12, 2022, the contents of which are incorporated by reference and constitute a part of this application.

### Reference Signs List

1 dispensing preparation apparatus
2 tank
3 pump unit
4 reservoir
5 pipe system
6 controller
7 dispensing head
8 plate exchanger
10 dispensing apparatus
21 bottle container
22 turntable
23 motor
51 tank pipe
52 reservoir pipe
53 pump pipe
54 valve

## Claims

1. A dispensing preparation apparatus comprising:
a reservoir configured to accommodate cell suspension, the reservoir having an opening, a discharge tip portion of a pipette repeatedly performing an entry and an exit through the opening, the pipette discharging the cell suspension to a cell accommodating portion;
a tank configured to accommodate the cell suspension;
a pipe connecting the tank and the reservoir, the pipe configured to allow the cell suspension to flow inside from the tank to the reservoir by a pump unit;
a turntable configured to rotate the tank about a rotation axis perpendicular to a bottom surface of the tank; and
a controller configured to control rotation of the turntable,
the pipe arranged such that an opening at one end of the pipe contacts an inner wall of the reservoir and an opening at another end of the pipe is spaced apart from an inner wall of the tank,
the controller controlling initial stirring to be performed, the initial stirring including initial inertial flow generation of the cell suspension followed by reverse flow generation, the initial inertial flow generation involving forward rotation of the turntable in a predetermined direction about the rotation axis a first number of times, the reverse flow generation involving reverse rotation of the turntable in a direction opposite to the predetermined direction a second number of times.

2. A dispensing preparation apparatus according to claim 1, wherein a center of the bottom surface of the tank and the rotation axis of the turntable are spaced apart.

3. A dispensing preparation apparatus according to claim 1, wherein, in the controller, the second number of times is equal to or less than the first number of times.

4. A dispensing preparation apparatus comprising:
a dispensing preparation apparatus according to claim 1 ;
a dispensing head configured to cause the entry and the exit of the pipette, and to pour the cell suspension into the cell accommodating portion of a container having the cell accommodating portion; and
a plate exchanger configured to move the container in which the cell suspension is not poured into the cell accommodating portion to a predetermined location, and to collect the container in which pouring of the cell suspension into the cell accommodating portion is completed.

5. A dispensing preparation apparatus according to claim 4, wherein, in the entry and the exit of the pipette, the dispensing head starts suction of the cell suspension after the front end of the pipette reaches a predetermined depth in a depth direction of the cell suspension from a surface of the cell suspension in the reservoir.

6. A dispensing preparation apparatus according to any one of claims 1 to 5, wherein, after the initial stirring, the controller performs additional stirring that includes additional inertial flow generation involving rotating the turntable by forward rotation in the predetermined direction and additional reverse flow generation involving rotating the turntable by reverse rotation in the direction opposite to the predetermined direction after the additional inertial flow generation.

7. A dispensing preparation apparatus according to claim 6, wherein a number of times of rotation of each rotation in the additional stirring is less than a number of times of rotation of each rotation in the initial stirring.

8. A dispensing preparation method of stirring cell suspension in a tank by a dispensing preparation apparatus, the dispensing preparation apparatus comprising:
a reservoir configured to accommodate the cell suspension, the reservoir having an opening, a discharge tip portion of a pipette repeatedly entering and exiting through the opening, the pipette discharging the cell suspension to a cell accommodating portion;
the tank configured to accommodate the cell suspension;
a pipe connecting the tank and the reservoir, the pipe configured to allow the cell suspension to flow inside from the tank to the reservoir by a pump unit; and
a turntable configured to rotate the tank about a rotation axis perpendicular to a bottom surface of the tank,
the dispensing preparation method comprising a step of performing initial stirring, the initial stirring including initial inertial flow generation of the cell suspension followed by reverse flow generation, the initial inertial flow generation involving forward rotation of the turntable in a predetermined direction about the rotation axis a first number of times, the reverse flow generation involving reverse rotation of the turntable in a direction opposite to the predetermined direction a second number of times.

9. A dispensing preparation method according to claim 8, wherein the second number of times is equal to or less than the first number of times.

10. A dispensing preparation method according to claim 8 or 9, comprising a step of performing additional stirring after the initial stirring, the additional stirring including additional inertial flow generation and additional reverse flow generation, the additional inertial flow generation involving rotating the turntable by forward rotation in the predetermined direction, the additional reverse flow generation involving rotating the turntable by reverse rotation in the direction opposite to the predetermined direction after the additional inertial flow generation.
